# EUROPÄISCHE PATENTSCHRIFT

(11) **EP 3 561 779 B1**
(45) Veröffentlichungstag und Bekanntmachung des Hinweises auf die Patenterteilung: **28.08.2024**
(21) Anmeldenummer: 18169880.4
(22) Anmeldetag: 27.04.2018
(51) Int. Cl.: G07C 5/08

(54) **SYSTEM ZUR ERFASSUNG PERSONENBEZOGENER UNFALLDATEN IN EINEM FAHRZEUG**
SYSTEM FOR DETECTING PERSONAL ACCIDENT DATA IN A VEHICLE
SYSTÈME DE DÉTECTION DE DONNÉES D'ACCIDENT À CARACTÈRE PERSONNEL DANS UN VÉHICULE

(43) Veröffentlichungstag der Anmeldung: 30.10.2019
(73) Patentinhaber: Rothenbühler, Rudolf, 8634 Hombrechtikon (CH); Bono, Caroline, 8634 Hombrechtikon (CH)
(72) Erfinder: BONO, Caroline, 8634 Hombrechtikon (CH)
(74) Vertreter: Leimgruber, Fabian Alfred Rupert

(56) Entgegenhaltungen:
- DE-A1- 102014 200 567
- DE-A1- 102015 208 358
- JP-A- 2009 069 885
- US-A1- 2008 195 261
- US-A1- 2010 060 734
- US-A1- 2010 219 944
- US-A1- 2012 105 635

## Beschreibung

Die vorliegende Erfindung bezieht sich auf ein System zur Erfassung personenbezogener Unfalldaten in einem Fahrzeug, auf ein Verfahren zum Erfassen personenbezogener Unfalldaten in einem Fahrzeug.

DE 102014200567 A1 beschreibt ein Verfahren zur Übertragung von Bild- und Audiodaten von einem Unfall eines Fahrzeugs an eine weiterverarbeitende Stelle, z.B. im Rahmen eines automatisch generierten Notrufs (eCall). Die übermittelten Daten können dann beispielsweise zur Beurteilung der Schwere von Sach- und Personenschäden ausgewertet werden.

US 2008/0195261 beschreibt ebenfalls ein fahrzeuginternes System, mit dem es möglich ist, im Falle eines Unfalls automatisch einen Notruf an eine Rettungsleitzentrale zu senden.

Die US 2010/060734 A1 und US 2010/219944 A1 beschreiben Fahrzeugunfallrekorder welche, insbesondere während eines Unfalls, Bilder von Fahrzeuginsassen mit einer Kamera erfassen.

Durch einen Verkehrsunfall kann ein Fahrzeugfahrer oder weitere Fahrzeuginsassen verschiedene Verletzungen davontragen. Besonders verletzungsanfällig sind bei einem Unfall, insbesondere auch einem Auffahrunfall, die Halswirbelsäule (aufgrund einer plötzlichen und unvorhergesehenen Bewegung des Kopfes gegenüber des fixierten Rumpfes), der Kopf selber sowie Thorax, aber auch der Rumpf und die Extremitäten. Die verschiedenen Schleuderbewegungen des Kopfes gegenüber dem Rumpf während des Unfalls beim Halten des Sicherheitsgurtes erfolgen beispielsweise innerhalb eines sehr kurzen Zeitraums im Bereich weniger Hundert Millisekunden. Die daraus resultierenden Verletzungen bzw. medizinischen Symptome werden als Schleudertrauma (auch HWS-Distorsion oder Peitschenhiebsyndrom) bezeichnet und können in ihrer Schwere von überdehnten Bändern bis hin zu Wirbelbrüchen oder tödlichen Verletzungen reichen.

Ungünstige Aufprallwinkel können aber auch dazu führen, dass die Halterfunktion des Sicherheitsgurtes versagt mit der Folge unbegrenzter Polytraumata, was dann bildlich nachgewiesen werden kann.

Die meisten der auftretenden Verletzungen sind jedoch in herkömmlichen bildgebenden medizinischen Verfahren, z.B. in der Röntgendiagnostik, nicht bzw. nur schwer darstellbar, was u.a. eine medizinische Diagnose und die Beweisführung bei Leistungsforderungen an Versicherungen aufgrund von Langzeitschäden erschweren kann.

Die Aufgabe der vorliegenden Erfindung besteht darin, ein alternatives bzw. verbessertes System zur Erfassung personenbezogener Unfalldaten in einem Fahrzeug bzw. ein Verfahren zum Erfassen personenbezogener Unfalldaten in einem Fahrzeug bereitzustellen, insbesondere von personenbezogenen Unfalldaten, die beim Erstellen einer medizinischen Diagnose berücksichtigt werden können und die eine wirklichkeitsgetreue Rekonstruktion und Abbildung der ganzen Unfallabfolge erlaubt.

Diese Aufgabe wird gelöst durch ein System zur Erfassung personenbezogener Unfalldaten gemäß Anspruch 1, ein Fahrzeug gemäß Anspruch 10 und ein Verfahren zum Erfassen personenbezogener Unfalldaten gemäß Anspruch 11. Dabei können die Verfahren auch durch die untenstehenden bzw. in den Unteransprüchen ausgeführten Merkmale des Systems und/oder des Fahrzeugs weitergebildet sein oder umgekehrt, bzw. die Merkmale des Systems und des Fahrzeugs bzw. der Verfahren können auch jeweils untereinander zur Weiterbildung genutzt werden.

Ein erfindungsgemäßes System zur Erfassung personenbezogener Unfalldaten in einem Fahrzeug umfasst zumindest eine Infrarotkamera zur Erfassung von Bilddaten zumindest eines Fahrzeuginsassens, wobei die Infrarotkamera als Hochgeschwindigkeitskamera ausgebildet ist, einen Ringspeicher zum Speichern der erfassten Bilddaten und eine Steuereinheit. Die Steuereinheit ist dazu ausgebildet, ein Unfalleintrittssignal zu empfangen und den Ringspeicher und/oder die Infrarotkamera so zu steuern, dass das Speichern der Bilddaten zu einem Endzeitpunkt beendet wird, wobei der Endzeitpunkt um ein vorab definiertes Zeitintervall nach dem Empfangen des Unfalleintrittssignals liegt. Die personenbezogenen Unfalldaten umfassen zumindest die in dem Ringspeicher gespeicherten Bilddaten.

Im Folgenden bezeichnet eine Hochgeschwindigkeitskamera eine Kamera, die dazu geeignet ist, eine große Anzahl an Bildern pro Sekunde (frames per second, fps) aufzunehmen, d.h. die eine große Aufnahmegeschwindigkeit aufweist, und so besonders kurzzeitige und/oder besonders schnell ablaufende Vorgänge aufzunehmen. Beispielsweise kann als Kamera eine Hochgeschwindigkeitskamera mit einer Aufnahmegeschwindigkeit von mindestens 700 fps, vorzugsweise mindestens 1000 fps verwendet werden.

Ein Ringspeicher ist ein digitaler Speicher mit vorgegebener Größe bzw. Speicherkapazität, in dem Daten, im vorliegenden Fall u.a. die von der Hochgeschwindigkeitskamera erfassten Bilddaten, kontinuierlich gespeichert werden. Ist die maximale Größe des Ringspeichers erreicht und der Ringspeicher voll, so wird das jeweils älteste gespeicherte Element überschrieben, sodass die Daten in einer Schleife, auch Loop genannt, gespeichert werden. Daher ist die graphische Darstellung des Speichers eine Ringform. Der Ringspeicher kann beispielsweise durch eine geeignete Software implementiert sein, mittels der das Speichern und Auslesen von Daten in einem digitalen Speicher entsprechend gesteuert wird.

Vorzugsweise ist die Größe des Ringspeichers ausreichend, um während eines Zeitintervalls von mehreren Sekunden, beispielsweise 10 s oder 20 s, die von der Hochgeschwindigkeitskamera aufgenommenen Bilder zu speichern, bevor diese wieder überschrieben werden. Die benötigte Speicherkapazität hängt somit sowohl von der gewünschten Speicherdauer (d.h. der gewünschten Dauer eines als Bilddaten in dem Ringspeicher zu speichernden Vorgangs) bzw. der gewünschten Aufnahmedauer (d.h. der gewünschten Dauer einer gefilmten und gespeicherten Videosequenz) ab, als auch von der Aufnahmegeschwindigkeit der Hochgeschwindigkeitskamera und der Größe (d.h. Datenmenge) der zu speichernden Bilddaten.

Mit dem erfindungsgemäßen System zur Erfassung personenbezogener Unfalldaten ist es beispielsweise möglich, einen Fahrzeuginsassen in einem Fahrzeug kontinuierlich zu filmen und die erfassten Bilddaten in dem Ringspeicher zu speichern. Aufgrund der dabei anfallenden großen Menge an Daten werden die im Regelfall nicht benötigten Bilddaten in dem Ringspeicher nach einem vorab bestimmten Zeitintervall, das u.a. abhängig von der Größe des Ringspeichers ist, durch neu erfasste Bilddaten überschrieben. Im Falle eines Unfalls wird das Speichern neuer Bilddaten nach einer festgelegten Zeit gestoppt, um zu verhindern, dass unfallrelevante Daten wieder überschrieben werden. Somit umfasst der Ringspeicher nach Beenden des Speichervorgangs unfallrelevante Daten, d.h. insbesondere Aufnahmen des Fahrzeuginsassens unmittelbar nach dem Unfall bzw. während des Unfallgeschehens.

Durch die große Aufnahmegeschwindigkeit der Kamera ist es beispielsweise möglich, für eine Auswertung der Bilddaten die im Wesentlichen innerhalb weniger hundert Millisekunden ablaufenden unfallbedingten Beschleunigungs- und Abbremsvorgänge des Körpers des Fahrzeuginsassens in Zeitlupe darzustellen und somit Rückschlüsse auf mögliche Verletzungen des Fahrzeuginsassens ziehen zu können, d.h. das Unfallgeschehen aus medizinischer Sicht zu analysieren (im Folgenden als medizinische Unfallanalyse bezeichnet). Der Ringspeicher sorgt dabei beispielsweise dafür, dass die zu analysierende Datenmenge nicht zu groß ist und dass nicht benötigte Daten überschrieben, d.h. nicht gespeichert werden bzw. dass in dem Ringspeicher im Wesentlichen lediglich solche Bilddaten gespeichert sind, die für eine medizinische und allenfalls auch juristische Unfallanalyse relevant sind.

Eine derartige medizinische Unfallanalyse anhand der personenbezogenen Unfalldaten kann beispielsweise eine Erstversorgung des Verunfallten (d.h. des zumindest einen Fahrzeuginsassens) an der Unfallstelle und/oder in einer medizinischen Einrichtung verbessern um dadurch beispielsweise medizinische Folgeschäden zumindest zu verringern. Auch kann die medizinische Unfallanalyse beispielsweise bei einer fortführenden medizinischen Behandlung des Verunfallten zu Rate gezogen werden und/oder um Forderungen gegenüber einer Versicherung, insbesondere auch bei Rechtsstreitigkeiten, begründen zu können.

Vorzugsweise ist das vorab definierte Zeitintervall in Abhängigkeit einer der Speicherkapazität des Ringspeichers entsprechenden Aufnahmedauer festgelegt, wobei insbesondere das vorab definierte Zeitintervall mindestens die Hälfte, vorzugsweise mindestens zwei Drittel, der Aufnahmedauer des Ringspeichers entspricht. Damit wird beispielsweise sichergestellt, dass die Bilddaten noch für einen ausreichenden Zeitraum nach dem Unfalleintrittszeitpunkt weiter erfasst und gespeichert werden und somit geeignete Bilddaten für eine spätere medizinische Unfallanalyse zur Verfügung stehen. Zudem sind dadurch auch Bilddaten in dem Ringspeicher erfasst, die zu einem Zeitpunkt unmittelbar vor dem Unfall erfasst wurden und somit beispielsweise Informationen über die Position des Fahrzeuginsassens, beispielsweise eine Drehung und/oder Neigung seines Kopfes, zum Unfalleintrittszeitpunkt umfassen. Diese Informationen können beispielsweise ebenfalls für eine spätere medizinische Unfallanalyse hinzugezogen werden.

Vorzugsweise umfasst das System weiter einen Sensor zum Bereitstellen des Unfalleintrittssignals, beispielsweise einen Sensor zum Erfassen eines Zustands des Airbags und/oder einen Beschleunigungssensor und/oder einen Geschwindigkeitssensor. Damit ist es beispielsweise möglich, das Unfalleintrittssignal sensorbasiert bereitzustellen.

Alternativ oder zusätzlich umfasst das System weiter eine Analyseeinheit zum Analysieren der erfassten Bilddaten und Bereitstellen des Unfalleintrittssignals in Abhängigkeit der analysierten Bilddaten. Dadurch ist es beispielsweise möglich, hohe Beschleunigungen und/oder Geschwindigkeiten des Fahrzeuginsassens als Erkennungsmuster für einen Unfall zu verwenden. Insbesondere können dadurch die für eine medizinische Unfallanalyse relevanten Bilddaten selbst als Auslöser zum Abbrechen des Speichervorgangs verwendet werden, was die Gefahr eines irrtümlichen Abbruchs des Speicherns von Bilddaten verringern kann, da das Unfalleintrittssignal hierbei unabhängig von Sensoren bereitgestellt werden kann.

Vorzugsweise umfassen die personenbezogenen Unfalldaten weiter medizinische Daten des zumindest einen Fahrzeuginsassens, wobei die medizinischen Daten vorzugsweise in einem zweiten Speicher des Systems gespeichert sind, und/oder die personenbezogenen Unfalldaten umfassen weiter Betriebsdaten des Fahrzeugs, wobei die Betriebsdaten vorzugsweise in dem Ringspeicher gespeichert sind. Die medizinischen Daten können beispielsweise Informationen zu Vorerkrankungen und/oder Diagnosen und/oder Medikation, zu Medikamentenunverträglichkeiten und/oder Allergien und/oder Vorverletzungen und/oder Implantaten und/oder durchgeführten medizinischen Eingriffen, etc. des zumindest einen Fahrzeuginsassens umfassen.

Durch das Bereitstellen medizinischer Daten des zumindest einen Fahrzeuginsassens ist es beispielsweise möglich, diese bei einer Erstbehandlung des verunfallten Fahrzeuginsassens zu berücksichtigen und damit die Erstbehandlung zu verbessern. Die Betriebsdaten des Fahrzeugs, auch als physikalische Werte des Fahrzeugs bezeichnet, während bzw. kurz vor dem Unfallgeschehen, beispielsweise die Geschwindigkeit oder Beschleunigung des Fahrzeugs, können beispielsweise ebenfalls für eine medizinische Unfallanalyse hinzugezogen werden.

Vorzugsweise ist die Kamera zur Erfassung von Bilddaten mehrerer Fahrzeuginsassen geeignet und/oder das System umfasst mehrere als Hochgeschwindigkeitskameras ausgebildete Kameras. Dadurch ist es beispielsweise möglich, personenbezogene Unfalldaten (insbesondere Bilddaten) mehrerer, vorzugsweise aller Insassen des Fahrzeugs zu erfassen und zu speichern. Zudem ist es beispielsweise möglich, zumindest einen Fahrzeuginsassen mit mehreren Hochgeschwindigkeitskameras zu filmen, vorzugsweise aus verschiedenen Blickwinkeln, um z.B. eine dreidimensionale Darstellung der Bewegung des Fahrzeuginsassens während des Unfalls generieren zu können und somit die medizinische Unfallanalyse weiter zu verbessern.

Das System umfasst weiter zumindest eine Beleuchtungsvorrichtung zum Beleuchten des zumindest einen Fahrzeuginsassens, wobei die Beleuchtungsvorrichtung vorzugsweise so von der Steuereinheit gesteuert wird, dass sie zumindest ab dem Empfangen des Unfalleintrittssignal bis zu dem Endzeitpunkt eingeschaltet ist. Durch die Beleuchtungsvorrichtung ist es beispielsweise möglich, Bilder mit guter Qualität zu erfassen und zu speichern, insbesondere auch bei schlechten Lichtverhältnissen wie z.B. nachts, und somit eine gute Auswertbarkeit der Bilder zu erzielen. Andererseits ist es durch die Beleuchtung beispielsweise möglich, die Auflösung der Hochgeschwindigkeitskamera, mit der die Bilder erfasst werden, zu reduzieren und so die zu speichernde Datenmenge zu verringern. Die Beleuchtung erfolgt erfindungsgemäß im Infrarot Lichtbereich und die Bilderfassung erfolgt durch zumindest einer Infrarotkamera welche als Hochgeschwindigkeitskamera ausgebildet ist.

Vorzugsweise umfasst das System weiter einen vorzugsweise geschützten zweiten Speicher, z.B. einen Festplattenspeicher und/oder eine SD-Karte und/oder einen Speicherstick, zum Speichern zumindest der in dem Ringspeicher gespeicherten Bilddaten und/oder das System umfasst weiter eine Übermittlungseinheit zum vorzugsweise kabellosen und/oder verschlüsselten Übermitteln der personenbezogenen Unfalldaten an eine externe Stelle und/oder an einen externen Speicher und/oder einen externen geschützten Server, insbesondere eine Cloud. Damit ist es beispielsweise möglich, die personenbezogenen Unfalldaten für eine medizinische Unfallanalyse bereitzustellen. Durch das Übermitteln der personenbezogenen Unfalldaten an eine externe Stelle können diese beispielsweise von einem Ersthelfer und/oder einem erstbehandelnden Arzt für eine verbesserte Beurteilung des medizinischen Zustands des zumindest einen Fahrzeuginsassens herangezogen werden. Durch das Speichern in einem internen Speicher des Systems können die personenbezogenen Unfalldaten beispielsweise für eine spätere medizinische Unfallanalyse herangezogen werden, beispielsweise durch einen erst- oder weiterbehandelnden Arzt und/oder einen Gutachter oder vor Ort von einem Ersthelfer ausgelesen werden. Das Verschlüsseln der Daten dient der Geheimhaltung bzw. dem Datenschutz und verhindert einen unautorisierten Zugriff auf die Daten.

Das System umfasst weiter eine Analyseeinheit zum Analysieren der erfassten Bilddaten, wobei die Analyseeinheit derart ausgebildet ist, ein Gesicht des zumindest einen Fahrzeuginsassens mit zumindest einem gespeicherten Gesicht zu vergleichen. Dadurch ist es beispielsweise möglich, einer Rettungsleitzentrale noch vor Eintreffen der Ersthelfer an dem Unfallort die Identität des zumindest einen Fahrzeuginsassens bereitzustellen und/oder anhand der Gesichts- bzw. Personenerkennung die korrekten medizinischen Daten bereitzustellen.

Ein erfindungsgemäßes Fahrzeug umfasst ein oben beschriebenes System zur Erfassung personenbezogener Unfalldaten. Damit ist es beispielsweise möglich, ein Fahrzeug bereitzustellen, mit dem die oben in Bezug auf das System beschriebenen Wirkungen ebenfalls erzielbar sind.

Ein erfindungsgemäßes Verfahren zum Erfassen personenbezogener Unfalldaten in einem Fahrzeug umfasst die Schritte: Erfassen von Bilddaten zumindest eines Fahrzeuginsassens mittels zumindest einer Infrarotkamera, wobei die Infrarotkamera als Hochgeschwindigkeitskamera ausgebildet ist, und wobei zumindest eine Beleuchtungsvorrichtung Licht im Infrarotbereich zum Beleuchten des zumindest einen Fahrzeuginsassen aussendet, Speichern der erfassten Bilddaten in einem Ringspeicher und Empfangen eines Unfalleintrittssignals mittels einer Steuereinheit, wobei die Steuereinheit den Ringspeicher und/oder die Infrarotkamera so steuert, dass das Speichern der Bilddaten zu einem Endzeitpunkt beendet wird, wobei der Endzeitpunkt um ein vorab definiertes Zeitintervall nach dem Empfangen des Unfalleintrittssignals liegt und wobei die personenbezogenen Unfalldaten zumindest die in dem Ringspeicher gespeicherten Bilddaten umfassen, und Analysieren der erfassten Bilddaten mit einer Analyseeinheit, wobei sie ein Gesicht des zumindest einen Fahrzeuginsassen mit zumindest einem gespeicherten Gesicht vergleicht. Damit sind die oben in Bezug auf das System beschriebenen Wirkungen beispielsweise auch mit einem Verfahren zum Erfassen personenbezogener Unfalldaten erzielbar.

Ein vorgeschlagenes Verfahren zum Erstellen einer medizinischen Diagnose zumindest eines Fahrzeuginsassens nach einem Unfall umfasst die Schritte: Bereitstellen von personenbezogenen Unfalldaten, die durch ein oben beschriebenes System und/oder Fahrzeug und/oder in einem oben beschriebenen Verfahren erfasst wurden, und Analysieren der personenbezogenen Unfalldaten durch einen Ersthelfer und/oder einen behandelnden Arzt und/oder einen Gutachter und/oder eine weitere fachkundige Person. Damit ist es beispielsweise möglich, die Bilddaten im Falle eines Unfalls zur sofortigen medizinischen Analyse zu analysieren um bereits im Voraus, insbesondere vor einer (Erst-)Untersuchung des Verunfallten, eine Einschätzung der körperlichen Verfassung und möglicher Verletzungen des Verunfallten vorzunehmen und/oder unterstützend zur Erstellung einer medizinischen Diagnose heranzuziehen, insbesondere in Verbindung mit einer medizinischen Untersuchung des Verunfallten, und/oder eine erstellte medizinische Diagnose im Nachhinein zu überprüfen und/oder gegebenenfalls zu korrigieren.

Vorzugsweise werden zum Erstellen der medizinischen Diagnose die von den personenbezogenen Unfalldaten umfassten Bilddaten derart analysiert, dass zumindest ein Bewegungsablauf eines Kopfes des zumindest einen Fahrzeuginsassens während des Unfalls erfasst wird, vorzugsweise als eine Relativbewegung, insbesondere in Relation zu einem Oberkörper und/oder Schulterbereich des zumindest einen Fahrzeuginsassens. Insbesondere wird der gesamte Körper mit Kopf, Hals, Thorax, Arme, Rumpf und allenfalls auf partielle Teile der unteren Extremitäten erfasst. Damit ist es beispielsweise möglich, Rückschlüsse auf unfallbedingte Verletzungendes Fahrzeuginsassens zu ziehen.

Weitere Merkmale und Zweckmäßigkeiten der Erfindung ergeben sich aus der Beschreibung von Ausführungsbeispielen anhand der beigefügten Zeichnungen.
Fig. 1a ist eine schematische Ansicht eines Fahrzeugs mit einem System gemäß einem ersten Ausführungsbeispiel der vorliegenden Offenbarung, welches in Fig. 1b schematisch gezeigt ist,
Fig. 2 zeigt schematisch ein Verfahren zum Erfassen und Analysieren personenbezogener Unfalldaten mittels dem in Fig. 1a und Fig. 1b gezeigten System,
Fig. 3 zeigt eine Zeitachse, die schematisch die Speicherdauer des Ringspeichers in Bezug auf einen Unfallerfassungszeitpunkt zeigt,
Fig. 4 ist eine schematische Ansicht eines Systems gemäß einem zweiten Ausführungsbeispiel der vorliegenden Offenbarung und
Fig. 5 ist eine schematische Ansicht eines Systems gemäß einem dritten Ausführungsbeispiel der vorliegenden Offenbarung.

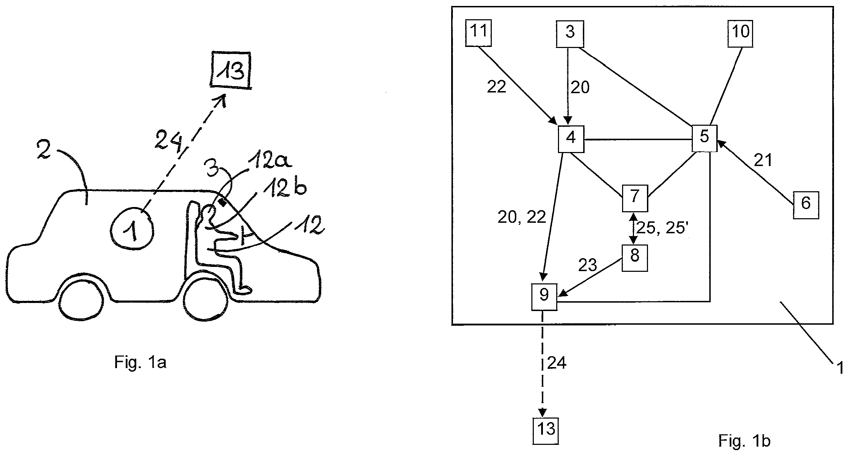

Im Folgenden wird mit Bezug auf Fig. 1a und 1b ein erstes Ausführungsbeispiel der vorliegenden Offenbarung beschrieben. Fig. 1a zeigt ein Fahrzeug 2 mit einem Fahrzeuginsassen 12 und einem schematisch dargestellten fahrzeuginternes System 1 zur Erfassung personenbezogener Unfalldaten, wobei das System 1 für eine übersichtlichere Darstellung schematisch in Fig. 1b gezeigt ist. Das in Fig. 1a beispielhaft gezeigte Fahrzeug 2 ist ein KFZ, es kann jedoch auch beispielsweise als ein Nutzfahrzeug ausgebildet sein.

Das fahrzeuginterne System 1 zur Erfassung personenbezogener Unfalldaten umfasst im Wesentlichen zumindest eine Kamera, die als eine Hochgeschwindigkeitskamera 3 ausgebildet ist, einen Ringspeicher 4, eine Steuereinheit 5 und eine Übermittlungseinheit 9.

Die Hochgeschwindigkeitskamera 3 des Systems 1 ist so ausgebildet und in dem Fahrzeug 2 angeordnet, dass sie dazu geeignet ist, einen Bereich des Fahrzeuginnenraums zu erfassen, in dem sich der Fahrzeuginsasse 12 befindet, d.h. eine Videosequenz in Form von Bildern des Fahrzeuginsassens 12 aufzunehmen. Dabei muss die Hochgeschwindigkeitskamera 3 nicht den gesamten Körper des Fahrzeuginsassens 12 erfassen, es ist vielmehr ausreichend, einen oberen Abschnitt dessen Körpers, insbesondere den Kopf 12a, den Hals und den Oberkörper und/oder Schulterbereich 12b, zu erfassen. Optimalerweise erfasst die Kamera auch distalere Körperbereiche

Die Hochgeschwindigkeitskamera 3 weist eine hohe Aufnahmegeschwindigkeit, beispielsweise von mindestens 700 fps, vorzugsweise mindestens 1000 fps auf. Die Bilder der Videosequenz werden von der Hochgeschwindigkeitskamera 3 als digitale Bilddaten 20 erfasst.

Der Ringspeicher 4 des Systems 1 ist an beliebiger Stelle des Fahrzeugs 2 vorgesehen und mittels einer Datenverbindung, beispielsweise einem Datenkabel, mit der Hochgeschwindigkeitskamera 3 verbunden und dazu geeignet, die von der Hochgeschwindigkeitskamera 3 erfassten Bilddaten 20 in digitaler Form zu speichern. Alternativ kann der Ringspeicher 4 auch ein interner Speicher der Hochgeschwindigkeitskamera 3 sein.

Der Ringspeicher 4 weist eine Speicherkapazität auf, die einer Aufnahmedauer T entspricht, d.h. von der Hochgeschwindigkeitskamera 3 erfasste und in dem Ringspeicher 4 gespeicherte Bilddaten 20 verbleiben für die Aufnahmedauer T in dem Ringspeicher 4, bevor sie durch neu erfasste Bilddaten 20 überschrieben werden. Die Speicherkapazität des Ringspeichers 4 kann beispielsweise so gewählt sein, dass sie einer Aufnahmedauer T von mindestens 10s, vorzugsweise mindestens 15, weiter bevorzugt mindestens 20s entspricht. Diese Werte verstehen sich lediglich als beispielhafte Angaben, die Aufnahmedauer kann 10s auch unterschreiten.

Weiter umfasst das System 1 einen in dem Fahrzeug 2 an geeigneter Stelle vorgesehenen Sensor 6, der dazu geeignet ist, einen Unfall des Fahrzeugs 2 festzustellen und ein entsprechendes Unfalleintrittssignal 21 zu erzeugen. Beispielsweise kann der Sensor 6 ein Airbagsensor sein, der einen Zustand eines Airbags (nicht gezeigt) des Fahrzeugs 2 erfasst und bei Aktivieren des Airbags ein Unfalleintrittssignal 21 erzeugt, und/oder der Sensor 6 kann ein Geschwindigkeits- oder Beschleunigungs- und/oder Rotationssensor sein, der bei einer Überschreitung einer vorab festgelegten Geschwindigkeitsänderung bzw. einer vorab festgelegten Beschleunigung bzw. einer vorab definierten Rotation ein Unfalleintrittssignal 21 erzeugt. Unter dem Begriff "Beschleunigung" sind auch negative Beschleunigungen, d.h. Abbremsvorgänge, zu verstehen.

Weiter umfasst das System 1 optional eine an geeigneter Stelle des Fahrzeugs 2 vorgesehene Betriebsdatenerfassungseinheit 11 zum Erfassen von Betriebsdaten 22 des Fahrzeugs 2, wobei die Betriebsdatenerfassungseinheit 11 mit dem Ringspeicher 4 über eine Datenverbindung, beispielsweise ein Datenkabel, verbunden ist zum Speichern der Betriebsdaten 22 in dem Ringspeicher 4, vorzugsweise in Verbindung mit den Bilddaten 20. Die Betriebsdatenerfassungseinheit kann beispielsweise als Geschwindigkeitssensor und/oder als Positionssensor, beispielsweise als GPS, ausgebildet sein zum Erfassen einer momentanen Geschwindigkeit bzw. Position des Fahrzeugs 2 als Betriebsdaten 22.

Optional umfasst das System 1 weiter eine Beleuchtungsvorrichtung 10 des Fahrzeugs 2, die derart ausgebildet und in dem Fahrzeug 2 angeordnet ist, dass sie zum Beleuchten des Fahrzeuginnenraums, insbesondere eines Bereichs des Fahrzeuginnenraums, in dem sich der Fahrzeuginsassen 12 befindet, geeignet ist.

Ferner umfasst das System 1 optional einen zweiten fahrzeuginternen Datenspeicher 8, in dem Gesichtserkennungsdaten 25' des zumindest einen Fahrzeuginsassens 12 und medizinische Daten 23 des zumindest einen Fahrzeuginsassens 12 gespeichert sind. Die medizinischen Daten 23 des Fahrzeuginsassens 12 können beispielsweise Informationen zu Medikamentenunverträglichkeiten und/oder Allergien und/oder Vorverletzungen und/oder Implantaten und/oder durchgeführten medizinischen Eingriffen umfassen. Der zweite Datenspeicher 8 kann beispielsweise als ein Festplattenspeicher und/oder eine SD-Karte und/oder ein Speicherstick ausgebildet sein.

Eine ebenfalls optionale fahrzeuginterne Analyseeinheit 7 des Systems 1 steht über jeweils eine Datenverbindung (z.B. ein Datenkabel) mit dem Ringspeicher 4 und dem zweiten Datenspeicher 8 in Verbindung und ist dazu geeignet, aus den in dem Ringspeicher 4 gespeicherten Bilddaten 20 Gesichtserkennungsdaten 25 zu berechnen und diese mit den in dem zweiten Datenspeicher 8 gespeicherten Gesichtserkennungsdaten 25' zu vergleichen. Hierzu kann die Analyseeinheit 7 eine CPU enthalten, deren Betrieb durch ein Computerprogramm (Software) zur Gesichtserkennung gesteuert wird.

Die Übermittlungseinheit 9 des Systems 1 ist ebenfalls an beliebiger Stelle in dem Fahrzeug 2 angeordnet und steht über jeweils eine Datenverbindung (z.B. Datenkabel) in Verbindung mit dem Ringspeicher 4 und dem zweiten Datenspeicher 8 des Systems 1. Sie ist dazu geeignet, die Bilddaten 20 sowie optional die Betriebsdaten 22 und medizinischen Daten 23 als personenbezogene Unfalldaten 24 mittels eines kabellosen Übermittlungsverfahrens an eine externe Stelle 13 vorzugsweise verschlüsselt zu übermitteln. Hierzu weist die Übermittlungseinheit 9 eine nicht gezeigte Datenschnittstelle auf.

Die externe Stelle 13 kann beispielsweise eine Rettungsleitzentrale sein und/oder ein Ersthelfer und/oder ein insbesondere erstbehandelnder Arzt bzw. eine insbesondere erstbehandelnde medizinische Einrichtung, z.B. ein Unfallklinikum. Die externe Stelle 13 kann auch ein fahrzeugexterner Datenspeicher sein, beispielsweise ein externer Server oder eine Cloud.

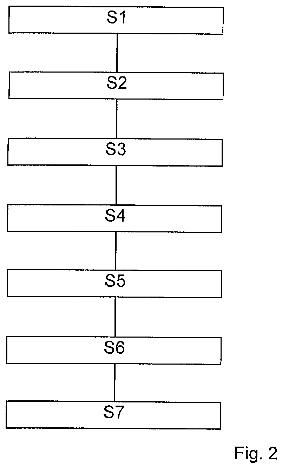

Die fahrzeuginterne Steuereinheit 5 des Systems 1 ist dazu ausgebildet, die einzelnen fahrzeuginternen Komponenten des Systems 1 zu steuern. Hierzu ist sie mit den jeweiligen Komponenten über Datenverbindungen (z.B. Datenkabel) verbunden. Die Steuereinheit 5 kann eine CPU enthalten, deren Betrieb durch ein Computerprogramm (Software) gesteuert wird. Insbesondere ist die Steuereinheit 5 dazu geeignet, das Unfalleintrittssignal 21 von dem Sensor 6 zu empfangen und daraufhin die Hochgeschwindigkeitskamera 3, den Ringspeicher 4, die Analyseeinheit 7 und die Übermittlungseinheit 9 so zu steuern, dass sie ein unten mit Bezug auf Fig. 2 beschriebenes Verfahren durchführen.

Das System 1 kann beispielsweise komplett oder teilweise als ein Aus- oder Nachrüstsatz für das Fahrzeug 2 bereitgestellt sein oder bereits in das Fahrzeug 2 integriert sein. Dabei können auch bereits in dem Fahrzeug 2 vorhandene Komponenten für das System 1 verwendet werden, beispielsweise kann der Sensor 6 ein bereits in dem Fahrzeug 2 vorhandener Sensor sein und/oder die Steuereinheit 5 kann eine bereits in dem Fahrzeug 2 vorhandene Steuereinheit sein, die durch ein geeignetes Computerprogramm (Software) zum Steuern der Komponenten des Systems 1 dient.

Fig. 2 zeigt schematisch ein Verfahren zum Erfassen personenbezogener Unfalldaten in dem mit Bezug auf Fig. 1a und 1b beschriebenem Fahrzeug 2. In Schritt S1 filmt die Hochgeschwindigkeitskamera 3 während der Fahrt kontinuierlich den Fahrzeuginsassen 12, d.h. erfasst Bilddaten 20 von dem Fahrzeuginsassen 12, und übermittelt diese an den Ringspeicher 4. Optional werden gleichzeitig durch die Betriebsdatenerfassungseinheit 11 kontinuierlich oder zu diskreten, vorzugsweise äquidistanten Zeitpunkten, während der Fahrt Betriebsdaten 22 des Fahrzeugs 2 erfasst und ebenfalls an den Ringspeicher 4 übermittelt.

Die Bilddaten 20 und Betriebsdaten 22 werden in Schritt S2 in dem Ringspeicher 4 gespeichert, wobei die Daten vorzugsweise so gespeichert werden, dass die zu einem Zeitpunkt erfassten Betriebsdaten 22 den zu dem jeweiligen Zeitpunkt erfassten Bilddaten 20 zugeordnet sind. Diese zeitliche Zuordnung der Betriebsdaten 22 und Bilddaten 20 kann beispielsweise durch eine Einbettung der Betriebsdaten 22 in die Bilddaten 20 erfolgen, z.B. indem die Betriebsdaten 22 als Metadaten der zeitlich zugeordneten Bilddaten 20 gespeichert werden oder graphisch in die Bilddaten 20 integriert werden, beispielsweise als Schriftzug. Aufgrund der begrenzten Speicherkapazität des Ringspeichers 4 sind die Bilddaten 20 und dazugehörigen Betriebsdaten 22 jeweils lediglich während der begrenzten Aufnahmedauer T in dem Ringspeicher 4 gespeichert und werden dann wieder von neuen Daten überschrieben.

Da der Ringspeicher 4 wie oben beschrieben eine begrenzte Speicherkapazität aufweist, kann es vorteilhaft sein, die Bilddaten 20 vor ihrer Speicherung in dem Ringspeicher 4 so zu bearbeiten, dass sie eine geringere Datenmenge umfassen. Beispielsweise können die den Bilddaten 20 entsprechenden Bildausschnitte verkleinert werden und/oder die von der Hochgeschwindigkeitskamera 3 aufgenommenen farbigen Bilder können beispielsweise als Graustufenbilder gespeichert werden. Hierzu kann eine geeignete, vorzugsweise in die Hochgeschwindigkeitskamera 3 integrierte Bildbearbeitungssoftware (nicht gezeigt) bereitgestellt sein.

In Schritt S3 wird durch den Sensor 6 ein Unfall des Fahrzeugs 2 erkannt, beispielsweise durch die Aktivierung eines Airbags und/oder eine signifikante Verringerung der Geschwindigkeit des Fahrzeugs 2 und/oder ein signifikantes Abbremsen (negative Beschleunigung) des Fahrzeugs 2 und/oder eine signifikante Rotation oder Neigung des Fahrzeugs 2. Daraufhin sendet der Sensor 6 ein Unfalleintrittssignal 21 an die Steuereinheit 5.

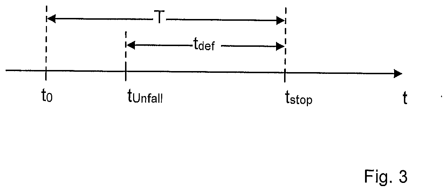

Die Steuereinheit 5 stoppt dann im Schritt S4 das Speichern der Bilddaten 20 und der Betriebsdaten 22 in dem Ringspeicher 4. Der Endzeitpunkt tₛₜₒₚ, zu dem das Speichern der Daten beendet wird, liegt um ein vorab definiertes Zeitintervall t_{def} nach dem Empfangen des Unfalleintrittssignals 21 zum Zeitpunkt t_{Unfall} durch die Steuereinheit 5, wie Fig. 3 zeigt. Das vorab definierte Zeitintervall t_{def} ist dabei höchstens so groß wie die der Speicherkapazität des Ringspeichers 4 entsprechende Aufnahmedauer T, d.h. t_{def} ≤ T, und entspricht vorzugsweise mindestens der Hälfte, weiter bevorzugt mindestens zwei Drittel der der Speicherkapazität des Ringspeichers 4 entsprechenden Aufnahmedauer T. Dadurch wird sichergestellt, dass die zum Unfallzeitpunkt und kurz danach (d.h. insbesondere während des Unfallgeschehens) erfassten Bilddaten 20 und Betriebsdaten 22 in dem Ringspeicher 4 nicht mehr überschrieben werden.

Somit liegen nach Beenden des kontinuierlichen Speicherns in dem Ringspeicher 4 gespeicherte Bilddaten 20 und Betriebsdaten 22 vor, die, entsprechende der der Speicherkapazität des Ringspeichers 5 entsprechenden Aufnahmedauer T, zwischen einem Zeitpunkt t₀, der zeitlich vor dem Zeitpunkt t_{Unfall} des Erfassens des Unfalleintrittssignals 21 durch die Steuereinheit 5 liegt, und dem Endzeitpunkt tₛₜₒₚ nach dem Erfassen des Unfalleintrittssignals erfasst wurden. Da der Zeitpunkt t_{Unfall} des Erfassens des Unfalleintrittssignals 21 als Unfallzeitpunkt angenommen werden kann, liegen somit Daten von einem Zeitraum kurz vor und kurz nach dem Unfall vor.

Bei einer Speicherkapazität des Ringspeichers 4, die einer Aufnahmedauer von T = 10s entspricht, kann das vorab definierte Zeitintervall beispielsweise t_{def} = 5s sein, so dass nach Beenden der Datenspeicherung in dem Ringspeicher 4 Bilddaten 20 und Betriebsdaten 22 gespeichert sind, die während eines Zeitraums von 5 Sekunden vor dem Unfall bis 5 Sekunden nach dem Unfall erfasst wurden. Bei einer Aufnahmedauer von T = 10s und einem vorab definierten Zeitintervall von beispielsweise t_{def} = 7s liegen nach Beenden der Datenspeicherung in dem Ringspeicher 4 Bilddaten 20 und Betriebsdaten 22 vor, die während eines Zeitraums von 3 Sekunden vor dem Unfall bis 7 Sekunden nach dem Unfall erfasst wurden.

Die hier genannten Zeitangaben für die Aufnahmedauer T und das vorab definierte Zeitintervall t_{def} sind lediglich als beispielhafte Werte zu verstehen, die Aufnahmedauer T und das vorab definierte Zeitintervall t_{def} können im Rahmen der Erfindung auch von den genannten Werten abweichen.

Das Beenden der Datenspeicherung kann beispielsweise dadurch erfolgen, dass die Steuereinheit 5 zu dem Endzeitpunkt tₛₜₒₚ einen Befehl zum Ausschalten der Hochgeschwindigkeitskamera 3 und der Betriebsdatenerfassungseinheit 11 an diese aussendet, so dass keine neuen Bilddaten 20 und Betriebsdaten 22 mehr erzeugt werden, und/oder dadurch, dass die Steuereinheit 5 den Speichervorgang selbst durch geeignetes Ansteuern des Ringspeichers 4 unterbricht.

Des Weiteren wird in dem Schritt S4 die Beleuchtungsvorrichtung 10 von der Steuereinheit 5 derart angesteuert, dass die Beleuchtungsvorrichtung 10 zumindest ab dem Empfangen des Unfalleintrittssignal zum Zeitpunkt t_{Unfall} bis zu dem Endzeitpunkt tₛₜₒₚ eingeschaltet ist und zumindest einen Bereich des Innenraums des Fahrzeugs 2 beleuchtet, in dem sich der von der Hochgeschwindigkeitskamera 3 erfasste Abschnitt des Körpers des Fahrzeuginsassens 12 befindet.

In dem anschließenden Schritt S5 werden die nach dem Beenden der Datenspeicherung in dem Ringspeicher 4 vorliegenden Bilddaten 20 durch die Analyseeinheit 7 beispielsweise mittels einer Gesichtserkennungssoftware analysiert. Die so erfassten Gesichtserkennungsdaten 25 werden mit den in dem zweiten Speicher 8 gespeicherten Gesichtserkennungsdaten 25' verglichen und bei ausreichender Übereinstimmung der beiden Datensätze, d.h. wenn das von der Hochgeschwindigkeitskamera 3 erfasste Gesicht des Fahrzeuginsassens 12 mit einer ausreichend hohen Wahrscheinlichkeit dem in dem Speicher 8 hinterlegten Gesicht entspricht, werden die in dem zweiten Speicher 8 gespeicherte medizinische Daten 23 der erkannten Person an die Übermittlungseinheit 9 übermittelt. Des Weiteren werden im Schritt S5 die nach dem Beenden der Datenspeicherung in dem Ringspeicher 4 vorliegenden Bilddaten 20 und Betriebsdaten 22 ebenfalls an die Übermittlungseinheit 9 übermittelt.

Die Gesichtserkennung kann auch unabhängig von dem Eintreten eines Unfalls durchgeführt werden, beispielsweise zu Beginn der Fahrt des Fahrzeugs 2 und/oder in regelmäßigen Intervallen während der Erfassung von Bilddaten 20.

Die in dem Ringspeicher 4 vorliegenden Bilddaten 20 und Betriebsdaten 22 werden dann in Schritt S6 zusammen mit den medizinischen Daten 23 als personenbezogene Unfalldaten 24 an die externe Stelle 13 übermittelt. Die Übermittlung der personenbezogenen Unfalldaten 24 erfolgt dabei kabellos, beispielsweise per Funk, insbesondere Mobilfunk, oder Internet, und kann beispielsweise zusammen mit einem elektronisch abgesetzten Notruf (ein sogenannter eCall) erfolgen. Vorzugsweise erfolgt das Übermitteln der personenbezogenen Unfalldaten 24 in verschlüsselter Form, beispielsweise mittels OpenPGP, wobei die personenbezogenen Unfalldaten 24 von der Übermittlungseinheit 9 mittels eines öffentlichen, von der externen Stelle 13 generierten Schlüssels verschlüsselt werden und nur durch den entsprechenden geheimen (privaten) Schlüssel der externen Stelle 13 wieder entschlüsselbar sind. Der öffentliche Schlüssel kann bereits in der Übermittlungseinheit 9 vorliegen, oder die Übermittlungseinheit 9 kann diesen vor der Verschlüsselung und Übermittlung der Daten von der externen Stelle 13 anfordern.

Anschließend werden im Schritt S7 die übermittelten und entschlüsselten personenbezogenen Unfalldaten 24 durch eine autorisierte und/oder entsprechend ausgebildete Person, beispielsweise einen Mitarbeiter der Rettungsleitzentrale und/oder einen Ersthelfer und/oder einen erstbehandelnden Arzt, ausgewertet, d.h. eine medizinische Unfallanalyse durchgeführt, beispielsweise um eine erste Abschätzung des Gesundheitszustandes des Fahrzeuginsassens 12 zu erhalten.

Insbesondere können die Bilddaten zum Erstellen einer medizinischen Diagnose derart ausgewertet werden, dass ein Bewegungsablauf idealerweise der ganze Körper, insbesondere der Kopf 12a des Fahrzeuginsassens 12 während des Unfalls erfasst wird. Insbesondere wird hierbei die Relativbewegung des Kopfes 12a in Relation zu dem Oberkörper und/oder Schulterbereich 12b des Fahrzeuginsassens analysiert um Rückschlüsse auf unfallbedingte Verletzungen im Bereich der Halswirbelsäule ziehen zu können.

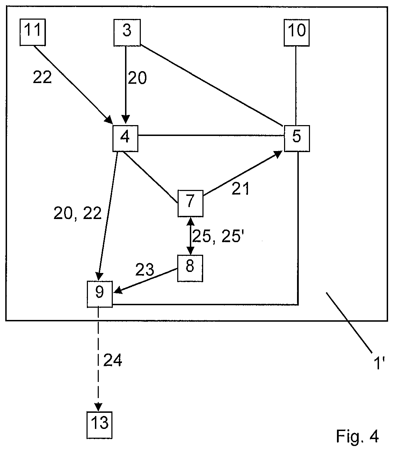

Fig. 4 zeigt ein fahrzeuginternes System 1' gemäß einem zweiten Ausführungsbeispiel der vorliegenden Offenbarung. Das in Fig. 4 gezeigte System 1' unterscheidet sich im Wesentlichen darin von dem in Fig. 1b gezeigten System 1, dass die mit dem Ringspeicher 4 über eine Datenverbindung in Verbindung stehenden Analyseeinheit 7 dazu ausgebildet ist, beispielsweise mittels eines geeigneten Bildverarbeitungsprogramms im Falle eines Unfalls des Fahrzeugs 2 anhand der in dem Ringspeicher 4 gespeicherten Bilddaten 20 ein Unfalleintrittssignal 21 zu erzeugen und dieses an die Steuereinheit 5 weiterzuleiten. Hierzu kann die Analyseeinheit 7 eine CPU enthalten, deren Betrieb durch ein Computerprogramm (Software) zur Bilderkennung gesteuert wird. Das Unfalleintrittssignal 21 kann beispielsweise bei einer von der Analyseeinheit 7 erkannten Bewegung des Fahrzeuginsassens 12, insbesondere einer einen vorab definierten Grenzwert überschreitenden Beschleunigung und/oder Geschwindigkeit des Fahrzeuginsassens 12, erzeugt werden.

Da das Unfalleintrittssignal 21 in dem in Fig. 4 gezeigten System 1' direkt aus den erfassten und gespeicherten Bilddaten 20 erzeugbar ist, wird für die Unfallerfassung der in Fig. 1b gezeigte Sensor 6 nicht benötigt.

Bei dem in Fig. 4 gezeigten System 1' wird das in Fig. 2 schematisch gezeigte Verfahren wird wie folgt abgewandelt: In Schritt S2 wird die Bilderkennung der in dem Ringspeicher 4 gespeicherten Bilddaten 20 durch die Analyseeinheit 7 kontinuierlich während der Fahrt des Fahrzeugs 2 durchgeführt. Erkennt die Analyseeinheit 7 anhand der analysierten Bilddaten 20 einen Unfall des Fahrzeugs 2, so übermittelt sie im Schritt S3 ein Unfalleintrittssignal 21 an die Steuereinheit 5. Die Schritte S1 und S4 bis S7 des mit Bezug auf Fig. 2 beschriebenen Verfahrens unterscheiden sich bei dem in Fig. 4 gezeigten System 1' nicht von denen des in Fig. 1b gezeigten Systems 1.

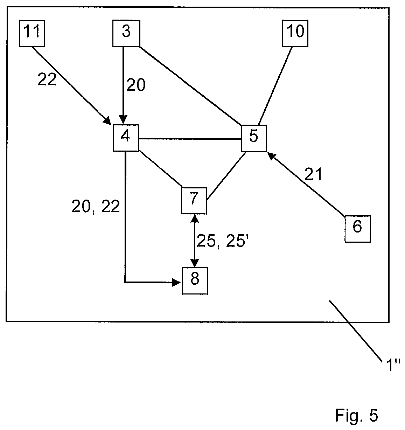

Fig. 5 zeigt ein fahrzeuginternes System 1" gemäß einem dritten Ausführungsbeispiel der vorliegenden Offenbarung. Das in Fig. 5 gezeigte System 1" unterscheidet sich im Wesentlichen darin von dem in Fig. 1b gezeigten System 1 und dem in Fig. 5 gezeigten System 1' dadurch, dass die personenbezogenen Unfalldaten 24 nicht mittels einer Übermittlungseinheit an eine externe Stelle übermittelt, sondern in dem fahrzeuginternen zweiten Datenspeicher 8 oder einem weiteren (nicht gezeigten) fahrzeuginternen Datenspeicher gespeichert werden. Um die personenbezogenen Unfalldaten 24 bzw. den zweiten Speicher 8 bei einem Unfall des Fahrzeugs 2 zu schützen, ist der zweite Speicher 8 vorzugsweise als ein geschützter Speicher (Blackbox) ausgebildet.

Bei dem mit Bezug auf Fig. 2 beschriebenen Verfahren werden bei dem in Fig. 5 gezeigten System 1" in Schritt S5 die Bilddaten 20 und optionalen Betriebsdaten 22 nicht an die Übermittlungseinheit, sondern an den fahrzeuginternen zweiten Datenspeicher 8, der bereits die optionalen medizinischen Daten 23 umfassen kann, übermittelt und in diesem gespeichert. Somit entfällt der in Fig. 2 gezeigte Schritt S6 des Übermittelns der Daten an eine externe Stelle. Zum Auswerten der personenbezogenen Unfalldaten 24 (Schritt S7) können diese beispielsweise aus dem zweiten Datenspeicher 8 ausgelesen bzw. heruntergeladen werden.

Die oben beschriebenen Merkmale der drei Ausführungsbeispiele können auch beliebig miteinander kombiniert werden. So kann beispielsweise das in Fig. 1b gezeigte System 1 und/oder das in Fig. 4 gezeigte System 1' anstelle der Übermittlungseinheit 9 lediglich den zweiten Datenspeicher 8 zur fahrzeuginternen Speicherung der personenbezogenen Unfalldaten 24 umfassen, wie in Bezug auf Fig. 5 beschrieben. Auch können die personenbezogenen Unfalldaten 24 sowohl in dem internen zweiten Speicher 8 gespeichert werden, als auch an die externe Stelle 13 übermittelt werden. Beispielsweise kann auch bei dem System 1 und/oder dem System 1", welches in Bezug auf Fig. 1b bzw. Fig. 5 beschrieben wurde, die Bereitstellung des Unfalleintrittssignals 21 durch Analysieren der Bilddaten 20 durch die Analyseeinheit 7 erfolgen (anstatt oder zusätzlich zur Bereitstellung des Unfalleintrittssignals 21 durch den Sensor 6) und/oder bei dem mit Bezug auf Fig. 4 beschriebenen System 1' kann ein zusätzliches Unfalleintrittssignal durch einen Sensor 6 bereitgestellt werden.

Die Betriebsdaten 22 und/oder medizinischen Daten 23 sind als optionale Daten in den personenbezogenen Unfalldaten 24 umfasst, beispielsweise um eine bessere medizinische Unfallanalyse bzw. eine bessere (Erst-)Versorgung des Verunfallten zu ermöglichen. Die personenbezogenen Unfalldaten 24 können auch lediglich die Bilddaten 20 umfassen. In diesem Fall kann das fahrzeuginterne System 1, 1', 1" zur Erfassung personenbezogener Unfalldaten 24 in dem Fahrzeug 2 auch ohne die Betriebsdatenerfassungseinheit 11 bzw. ohne den internen zweiten Datenspeicher 8 (falls das System eine Übermittlungseinheit 9 umfasst) bereitgestellt sein. Ferner kann das System 1, 1', 1" zur Erfassung personenbezogener Unfalldaten 24 auch ohne die Analyseeinheit 7 und/oder ohne die Beleuchtungsvorrichtung 10 bereitgestellt sein. Insbesondere kann die Analyse der erfassten Bilddaten 20 statt durch die Analyseeinheit 7 auch durch die Steuereinheit 5 selbst erfolgen.

Ferner können die Komponenten des fahrzeuginternen Systems 1, 1', 1" auch bereits in dem Fahrzeug 2 für eine andere Nutzung vorgesehene Komponenten sein und/oder die Komponenten des Systems 1, 1', 1" können für weitere Aufgaben und/oder Funktionen verwendet werden, d.h. die Komponenten können eine Mehrfachnutzung erfahren. Beispielsweise kann die Hochgeschwindigkeitskamera 3 auch einem Assistenz- und/oder Sicherheitssystem des Fahrzeugs 2 zugeordnet sein, beispielsweise um einen Sekundenschlaf des Fahrzeuginsassens 12 zu erkennen, und/oder der Sensor 6 und/oder die Betriebsdatenerfassungseinheit 11 können bereits in dem Fahrzeug 2 vorgesehene Komponenten sein.

Um die Beschreibung der Ausführungsbeispiele zu vereinfachen, sind diese lediglich in Bezug auf einen Fahrzeuginsassen ausgeführt. Das fahrzeuginterne System kann auch zur Erfassung personenbezogener Unfalldaten mehrerer Fahrzeuginsassen geeignet sein. Hierzu sind vorzugsweise mehrere Hochgeschwindigkeitskameras zur Erfassung von Bilddaten der Fahrzeuginsassen vorgesehen und/oder mehrere Ringspeicher zur Speicherung zumindest der Bilddaten.

Zudem kann auch ein Fahrzeuginsasse von mehr als einer Hochgeschwindigkeitskamera vorzugsweise aus verschiedenen Blickwinkeln gefilmt werden um beispielsweise eine dreidimensionale Ansicht des Fahrzeuginsassens zu generieren.

## Patentansprüche

1. System zur Erfassung personenbezogener Unfalldaten (24) in einem Fahrzeug (2), umfassend
zumindest eine Infrarotkamera zur Erfassung von Bilddaten (20) zumindest eines Fahrzeuginsassen (12), wobei die Infrarotkamera als Hochgeschwindigkeitskamera (3) ausgebildet ist,
einen Ringspeicher (4) zum Speichern der erfassten Bilddaten (20),
eine Steuereinheit (5), die dazu ausgebildet ist, ein Unfalleintrittssignal (21) zu empfangen und den Ringspeicher (4) und/oder die Infrarotkamera (3) so zu steuern, dass das Speichern der Bilddaten (20) zu einem Endzeitpunkt (*tₛₜₒₚ*) beendet wird, wobei der Endzeitpunkt (*tₛₜₒₚ*) um ein vorab definiertes Zeitintervall (*t_{def}*) nach dem Empfangen des Unfalleintrittssignals (21) liegt, und wobei die personenbezogenen Unfalldaten (24) zumindest die in dem Ringspeicher (4) gespeicherten Bilddaten (20) umfassen,
zumindest eine Beleuchtungsvorrichtung (10), die Licht im Infrarotbereich sendet, zum Beleuchten des zumindest einen Fahrzeuginsassen (12), und
eine Analyseeinheit (7) zum Analysieren der erfassten Bilddaten (20), wobei die Analyseeinheit (7) derart ausgebildet ist, ein Gesicht des zumindest einen Fahrzeuginsassen (12) mit zumindest einem gespeicherten Gesicht zu vergleichen.

2. System nach Anspruch 1, wobei das vorab definierte Zeitintervall (*t_{def}*) in Abhängigkeit einer Speicherkapazität des Ringspeichers (4) entsprechenden Aufnahmedauer (*T*) festgelegt ist, insbesondere wobei das vorab definierte Zeitintervall (*t_{def}*) mindestens die Hälfte, vorzugsweise mindestens zwei Drittel, der der Speicherkapazität des Ringspeichers (4) entsprechenden Aufnahmedauer (T) des Ringspeichers (4) entspricht.

3. System nach Anspruch 1 oder 2, weiter umfassend einen Sensor (6) zum Bereitstellen des Unfalleintrittssignals (21).

4. System nach einem der Ansprüche 1 bis 3, weiter umfassend eine Analyseeinheit (7) zum Analysieren der erfassten Bilddaten (20) und Bereitstellen des Unfalleintrittssignals (21) in Abhängigkeit der analysierten Bilddaten (20).

5. System nach einem der Ansprüche 1 bis 4, wobei die personenbezogenen Unfalldaten (24) weiter medizinische Daten (23) des zumindest einen Fahrzeuginsassen (12) umfassen, wobei die medizinischen Daten (23) vorzugsweise in einem zweiten Speicher (8) des Systems (1, 1', 1") gespeichert sind.

6. System nach einem der Ansprüche 1 bis 5, wobei die personenbezogenen Unfalldaten (24) weiter Betriebsdaten (22) des Fahrzeugs (2) umfassen, wobei die Betriebsdaten (22) vorzugsweise in dem Ringspeicher (4) gespeichert sind.

7. System nach einem der Ansprüche 1 bis 6, wobei die Kamera zur Erfassung von Bilddaten (20) mehrerer Fahrzeuginsassen (12) geeignet ist und/oder wobei mehrere als Hochgeschwindigkeitskameras (3) ausgebildete Kameras bereitgestellt sind.

8. System nach einem der Ansprüche 1 bis 7, wobei die Beleuchtungsvorrichtung (10) so von der Steuereinheit (5) gesteuert wird, dass sie zumindest ab dem Empfangen des Unfalleintrittssignal (21) bis zu dem Endzeitpunkt (*tₛₜₒₚ*) eingeschaltet ist.

9. System nach einem der Ansprüche 1 bis 8, weiter umfassend einen zweiten, vorzugsweise geschützten Speicher (8) zum Speichern zumindest der in dem Ringspeicher (4) gespeicherten Bilddaten (20) und/oder eine Übermittlungseinheit (9) zum vorzugsweise kabellosen Übermitteln der personenbezogenen Unfalldaten (24) an eine externe Stelle (13) und/oder an einen externen Speicher.

10. Fahrzeug, umfassend ein System (1, 1', 1") zur Erfassung personenbezogener Unfalldaten (24) nach einem der Ansprüche 1 bis 9.

11. Verfahren zum Erfassen personenbezogener Unfalldaten (24) in einem Fahrzeug (2) mit den Schritten:
Erfassen (S1) von Bilddaten zumindest eines Fahrzeuginsassen (12) mittels zumindest einer Infrarotkamera, wobei die Infrarotkamera als Hochgeschwindigkeitskamera (3) ausgebildet ist, und wobei zumindest eine Beleuchtungsvorrichtung (10) Licht im Infrarotbereich zum Beleuchten des zumindest einen Fahrzeuginsassen (12) aussendet,
Speichern (S2) der erfassten Bilddaten (20) in einem Ringspeicher (4) und
Empfangen (S4) eines Unfalleintrittssignals (21) mittels einer Steuereinheit (5),
wobei die Steuereinheit (5) den Ringspeicher (4) und/oder die Kamera (3) so steuert,
dass das Speichern der Bilddaten (20) zu einem Endzeitpunkt (*tₛₜₒₚ*) beendet wird, wobei der Endzeitpunkt (*tₛₜₒₚ*) um ein vorab definiertes Zeitintervall (*t_{def}*) nach dem Empfangen des Unfalleintrittssignals (21) liegt und wobei die personenbezogenen Unfalldaten (24) zumindest die in dem Ringspeicher (4) gespeicherten Bilddaten (20) umfassen, und
Analysieren (S5) der erfassten Bilddaten (20) mit einer Analyseeinheit (7), wobei sie ein Gesicht des zumindest einen Fahrzeuginsassen (12) mit zumindest einem gespeicherten Gesicht vergleicht.

## Claims

1. System for recording personal accident data (24) in a vehicle (2), comprising
at least one infrared camera for recording image data (20) of at least one vehicle occupant (12), wherein the infrared camera is designed as a high-speed camera (3),
a ring buffer (4) for storing the captured image data (20),
a control unit (5) which is designed to receive an accident entry signal (21) and to control the ring buffer (4) and/or the infrared camera (3) in such a way that the storage of the image data (20) is terminated at an end time (*tₛₜₒₚ*), wherein the end time (*tₛₜₒₚ*) is a predefined time interval (*t_{def}*) after the reception of the accident entry signal (21), and wherein the personal accident data (24) comprises at least the image data (20) stored in the ring buffer (4),
at least one illumination device (10) which emits light in the infrared range for illuminating the at least one vehicle occupant (12), and
an analysis unit (7) for analyzing the captured image data (20), wherein the analysis unit (7) is designed to compare a face of the at least one vehicle occupant (12) with at least one stored face.

2. System according to claim 1, wherein the predefined time interval (*t_{def}*) is determined as a function of a recording duration (*T*) corresponding to a storage capacity of the ring buffer (4), in particular wherein the predefined time interval (*t_{def}*) corresponds to at least half, preferably at least two thirds, of the recording duration (*T*) of the ring buffer (4) corresponding to the storage capacity of the ring buffer (4).

3. System according to claim 1 or 2, further comprising a sensor (6) for providing the accident entry signal (21).

4. System according to any one of claims 1 to 3, further comprising an analysis unit (7) for analyzing the captured image data (20) and providing the accident occurrence signal (21) depending on the analyzed image data (20).

5. System according to one of claims 1 to 4, wherein the personal accident data (24) further comprise medical data (23) of the at least one vehicle occupant (12), wherein the medical data (23) are preferably stored in a second memory (8) of the system (1, 1', 1").

6. System according to one of claims 1 to 5, wherein the personal accident data (24) further comprise operating data (22) of the vehicle (2), wherein the operating data (22) are preferably stored in the ring buffer (4).

7. System according to one of claims 1 to 6, wherein the camera is suitable for capturing image data (20) of several vehicle occupants (12) and/or wherein several cameras designed as high-speed cameras (3) are provided.

8. System according to one of claims 1 to 7, wherein the lighting device (10) is controlled by the control unit (5) such that it is switched on at least from the reception of the accident entry signal (21) until the end time (*tₛₜₒₚ*).

9. System according to one of claims 1 to 8, further comprising a second, preferably protected memory (8) for storing at least the image data (20) stored in the ring buffer (4) and/or a transmission unit (9) for transmitting the personal accident data (24), preferably wirelessly, to an external location (13) and/or to an external memory.

10. Vehicle, comprising a system (1, 1', 1") for recording personal accident data (24) according to one of claims 1 to 9.

11. method for recording personal accident data (24) in a vehicle (2), comprising the steps of:
capturing (S1) of image data of at least one vehicle occupant (12) by means of at least one infrared camera, wherein the infrared camera is designed as a high-speed camera (3), and wherein at least one illumination device (10) emits light in the infrared range for illuminating the at least one vehicle occupant (12),
storing (S2) the captured image data (20) in a ring buffer (4) and
receiving (S4) an accident occurrence signal (21) by means of a control unit (5), wherein the control unit (5) controls the ring buffer (4) and/or the camera (3) in such a way that the storage of the image data (20) is terminated at an end time (*tₛₜₒₚ*), wherein the end time (*tₛₜₒₚ*) is a predefined time interval (*t_{def}*) after the receipt of the accident entry signal (21) and wherein the personal accident data (24) comprises at least the image data (20) stored in the ring buffer (4), and
analyzing (S5) the captured image data (20) with an analysis unit (7),
wherein it compares a face of the at least one vehicle occupant (12) with at least one stored face.

## Revendications

1. Système d'enregistrement de données personnelles d'accident (24) dans un véhicule (2), comprenant
au moins une caméra infrarouge pour la saisie de données d'image (20) d'au moins un occupant du véhicule (12), la caméra infrarouge étant conçue comme une caméra à grande vitesse (3),
une mémoire annulaire (4) pour stocker les données d'image capturées (20),
une unité de commande (5) qui est conçue pour recevoir un signal d'entrée d'accident (21) et pour commander la mémoire annulaire (4) et/ou la caméra infrarouge (3) de telle sorte que l'enregistrement des données d'image (20) soit terminé à un moment final (*tₛₜₒₚ*), l'instant final (*tₛₜₒₚ*) se situant, d'un intervalle de temps (*t_{def}*) défini au préalable, après la réception du signal d'entrée d'accident (21), et les données d'accident (24) relatives aux personnes comprenant au moins les données d'image (20) enregistrées dans la mémoire annulaire (4),
au moins un dispositif d'éclairage (10) qui émet de la lumière dans le domaine infrarouge pour éclairer ledit au moins un occupant du véhicule (12), et
une unité d'analyse (7) pour analyser les données d'image (20) détectées, l'unité d'analyse (7) étant conçue de manière à comparer un visage du au moins un occupant (12) du véhicule avec au moins un visage mémorisé.

2. Système selon la revendication 1, dans lequel l'intervalle de temps (tdef) défini au préalable est fixé en fonction d'une durée d'enregistrement (T) correspondant à une capacité de mémoire de la mémoire annulaire (4), en particulier dans lequel l'intervalle de temps (*t_{def}*) défini au préalable correspond au moins à la moitié, de préférence au moins aux deux tiers, de la durée d'enregistrement (7) de la mémoire annulaire (4) correspondant à la capacité de mémoire de la mémoire annulaire (4).

3. Système selon la revendication 1 ou 2, comprenant en outre un capteur (6) pour fournir le signal d'entrée d'accident (21).

4. Système selon l'une des revendications 1 à 3, comprenant en outre une unité d'analyse (7) pour analyser les données d'image (20) acquises et fournir le signal d'entrée d'accident (21) en fonction des données d'image (20) analysées.

5. Système selon l'une des revendications 1 à 4, dans lequel les données d'accident (24) à caractère personnel comprennent en outre des données médicales (23) de l'au moins un occupant (12) du véhicule, les données médicales (23) étant de préférence stockées dans une deuxième mémoire (8) du système (1, 1', 1").

6. Système selon l'une des revendications 1 à 5, dans lequel les données d'accident (24) relatives à la personne comprennent en outre des données de fonctionnement (22) du véhicule (2), les données de fonctionnement (22) étant de préférence stockées dans la mémoire annulaire (4).

7. Système selon l'une des revendications 1 à 6, dans lequel la caméra est adaptée à la saisie de données d'image (20) de plusieurs occupants du véhicule (12) et/ou dans lequel plusieurs caméras conçues comme des caméras à grande vitesse (3) sont mises à disposition.

8. Système selon l'une des revendications 1 à 7, dans lequel le dispositif d'éclairage (10) est commandé par l'unité de commande (5) de manière à être allumé au moins à partir de la réception du signal d'entrée d'accident (21) jusqu'à l'instant final (*tₛₜₒₚ*)*.*

9. Système selon l'une des revendications 1 à 8, comprenant en outre une deuxième mémoire (8), de préférence protégée, pour stocker au moins les données d'image (20) stockées dans la mémoire annulaire (4) et/ou une unité de transmission (9) pour transmettre, de préférence sans câble, les données d'accident (24) relatives aux personnes à un service externe (13) et/ou à une mémoire externe.

10. Véhicule comprenant un système (1, 1', 1") de collecte de données personnelles d'accident (24) selon l'une quelconque des revendications 1 à 9.

11. Procédé de saisie de données d'accident (24) relatives à une personne dans un véhicule (2), comprenant les étapes suivantes :
détecter (S1) de données d'image d'au moins un occupant (12) du véhicule au moyen d'au moins une caméra infrarouge, la caméra infrarouge étant conçue comme une caméra à grande vitesse (3), et au moins un dispositif d'éclairage (10) émettant de la lumière dans le domaine infrarouge pour éclairer l'au moins un occupant (12) du véhicule,
stocker (S2) des données d'image (20) acquises dans une mémoire annulaire (4) et
recevoir (S4) d'un signal d'entrée d'accident (21) au moyen d'une unité de commande (5), l'unité de commande (5) commandant la mémoire annulaire (4) et/ou la caméra (3) de telle sorte que l'enregistrement des données d'image (20) soit terminé à un moment final (*tₛₜₒₚ*), l'instant final (*tₛₜₒₚ*) se situant, d'un intervalle de temps (*t_{def}*) défini au préalable, après la réception du signal d'entrée d'accident (21) et les données d'accident (24) relatives aux personnes comprenant au moins les données d'image (20) enregistrées dans la mémoire annulaire (4), et
analyser (S5) les données d'image acquises (20) avec une unité d'analyse (7), en comparant un visage du au moins un occupant (12) du véhicule avec au moins un visage mémorisé.
